# EUROPEAN PATENT APPLICATION

(11) **EP 4 578 376 A1**
(43) Date of publication of application: **02.07.2025**
(21) Application number: 23307401.2
(22) Date of filing: 27.12.2023
(51) Int. Cl.: A61B 5/00, G01N 21/17

(54) **A METHOD FOR ESTIMATING A LOCAL WAVELENGTH OF A WAVE WITHIN A SAMPLE SUBMITTED TO A PERIODIC MECHANICAL STRESS OR TO A PERIODIC THERMAL MODULATION**

(71) Applicant: Université de Strasbourg, 67000 Strasbourg (FR); Centre National de la Recherche, 75016 Paris (FR)
(72) Inventor: Nahas, Amir, 67100 Strasbourg (FR); Martins Seromenho, Emmanuel, 91270 Vigneux-sur-Seine (FR)
(74) Representative: Gevers & Orès

(57) **Abstract**

The invention relates to a method for estimating a local wavelength within a sample submitted to a periodic mechanical stress, comprising the following steps:
A) acquiring at least N images of the sample by full-field coherent optical imaging, where N ≥ 3,
for each integer k from 1 to N, acquiring a k^{th} image of the sample while said sample is submitted to said periodic mechanical stress as follow:
A1) launching acquisition of the k^{th} image,
A2) concurrently to the launching of acquisition of the k^{th} image, generating a periodic mechanical stress, of period T, on the sample,
A3) with a given time delay τₖ = τ₁ + (k-1)*τ_{offset} with respect to the launching of acquisition of the k^{th} image where τ_{offset} is a time offset, illuminating said sample with a series of periodic pulses, of period T,
A4) stopping acquisition of the k^{th} image after an acquisition time t_{ACQ} = n*T, where n ≥ 2,

B) estimating the local wavelength by using the N images previously acquired.

## Description

### Technical field of the invention

The invention relates to the field of estimation of a local wavelength of a wave within a sample. The invention more specifically relates to such an estimation by full-field coherent imaging.

### Prior art

The mechanical response of biological tissues has been studied extensively in recent decades. Indeed, mechanical properties are significantly associated with the pathological states of biological tissues. To this end, wave propagation velocity has been widely used in ultrasonic shear wave elastography (SWE) and magnetic resonance elastography (MRE) to assess the elastic and viscoelastic mechanical properties of human soft tissues.

For example, the paper of Kabir, I. E. et al. titled "Reverberant magnetic resonance elastographic imaging using a single mechanical driver", Phys. Med. Biol. 68, 055015 (2023) proposes a MRE technique. It highlights an elastography technique based on the reverberation of mechanical waves. Reverberant elastography is an approach that exploits the physical properties of diffuse fields. Reverberant shear wave fields are produced when multiple sources and reflections establish a three-dimensional wave field within an organ. This allows them to reconstruct a map of stiffness in the lateral direction, and along the depth axis. The aim of this paper is to show the feasibility of applying reverberant elastography with a single actuator and the use of a magnetic resonance imaging (MRI) system. In practice, MRI methods cannot be considered as real time techniques.

Full-field coherent optical imaging techniques provide high resolution, non-contact, non-invasive imaging of tissue displacements at the micron scale. The full-field coherent optical imaging techniques usually used are (i) speckle imaging, (ii) digital holography or (iii) full-field optical coherence tomography. Optical imaging techniques provide direct access to the phase and have a sensitivity of a few nanometers, which is particularly suitable for monitoring wave propagation, especially in biological tissues even on a cellular scale.

However, full-field optical imaging techniques generally have a reduced field of view compared with SWE and MRE techniques. As a result, tracking wave propagation can be challenging with these optical techniques because if the wavelength is often larger than the field of view or if the wave is travelling at high speed, it is difficult or impossible to track its propagation.

One solution to this problem is to use a stroboscopic approach that allows the propagation of periodic waves to be followed. This approach requires either modulation of the light source or modulation of the camera. In both cases, however, the luminous flux must be high enough to fill the pixels during a short camera acquisition time. The higher the frequency of the wave to be observed, the shorter the camera exposure time. This requires the use of high-power light sources or of a very sensitive camera to get a sufficient Signal to Noise Ratio (SNR) during a short exposure time.

For example, the paper of De Greef & al. "Digital Stroboscopic Holography Setup for Deformation Measurement at Both Quasi-Static and Acoustic Frequencies", International Journal of Optomechatronics 8, 275-291 (2014) presents a digital stroboscopic holography device (namely stroboscopy applied to (ii) digital holography). The authors of this paper use this method to identify and visualize complex vibrational patterns using a high-energy pulsed laser to record holograms in a single acquisition. The system illuminates a periodically moving object with a very short pulse, synchronized to a single phase of the period of movement. The illumination is then shifted in time to another phase of the period of movement and this process is repeated until the entire phase of the wave is followed. Synchronization is managed by an electronic module, which controls the illumination, camera, and actuator (vibrator). Accordingly, all the elements are synchronized via the electronic module. In addition, the authors use a high-power light source (to get sufficient SNR during the exposure time), which in practice limits their approach in terms of exposure time to illumination power ratio.

The prior arts discussed above are dedicated to a mechanical stress but it should be noted that the same kind of comments can be made with a thermal modulation of biological tissues or other type of samples for which we try to determine a local wavelength value.

### Summary of the invention

An aim of the invention is to overcome at least one of the aforementioned drawbacks.

More specifically, an aim of the invention is to propose a method for estimating a local wavelength of a wave within a sample submitted to a periodic mechanical stress or to a periodic thermal modulation, in enlarged frequency and speed domains.

To reach at least one of this aim, the invention proposes a method for estimating a local wavelength of a wave within a sample submitted to a periodic mechanical stress or to a periodic thermal modulation, comprising the following steps:
A) acquiring at least N images of the sample by full-field coherent optical imaging, where N is an integer equal or greater than three,
   for each integer k from 1 to N, acquiring a k^{th} image of the sample while said sample is submitted to said periodic mechanical stress or said periodic thermal modulation as follow:
   A1) launching acquisition of the k^{th} image,
   A2) concurrently to the launching of acquisition of the k^{th} image of step A1), generating a periodic mechanical stress or a periodic thermal modulation, of period T, on the sample,
   A3) with a given time delay τₖ = τ₁ + (k-1)*τ_{offset} with respect to the launching of acquisition of the k^{th} image of step A1) where τ_{offset} is a time offset, illuminating said sample with a series of periodic pulses, of period T, and
   A4) stopping acquisition of the k^{th} image after an acquisition time t_{ACQ} = n*T, where n is an integer greater or equal to 2,
B) estimating the local wavelength by using the N images acquired at step A).

Additional features of the method are as follow:
- an acquisition time for an image, defined between A4) and A1), is the same for each of the N images,
- launching of acquisition at steps A1) between the N images is periodic, with a period T₀,
- the mechanical stress or the thermal modulation is sinusoidal,
- step A) is carried out for N = 3 or 4 images,
- the mechanical stress is a shear stress,
- step B) comprises the following steps: B1) making a temporal correlation of a point of the sample with its neighbours over the N images acquired at step A, B2) making a derivation or a fit of the correlation function obtained at step B1) at least in space, B3) estimating the local wavelength thanks to the results of derivation or fit of step B2),
- the wave is a wave propagating within the sample, a stationary wave or a guided wave such as a Rayleigh or Lamb wave,
- acquisition of the N images is carried out with a 2D camera, for example of CMOS type,
- the camera has its own image acquisition time, and preferably its own periodicity, the periodic mechanical stress is generated by a piezoelectric actuator in contact with the sample together with a first low-frequency generator connected to said piezoelectric actuator and configured to generate a sinusoidal signal of period T,
- the low-frequency generator is connected to the camera,
- illumination is carried out by a Light-Emitting Diode,
- the series of periodic pulses is generated by a second low-frequency generator connected to the Light-Emitting Diode,
- the time delays are generated by a microcontroller both connected to the camera and to the second low-frequency generator.

The invention will be better understood with the help of the description that follows only provided as an example and carried out by reference to the annexed drawings in which:
- Figure 1 is a block diagram of the main steps of the method of the invention.
- Figure 2 represents an example of a device to implement the method of the invention.
- Figure 3 represents schematically the device of figure 2 together with the main electronic components to make it work.
- Figure 4 represents the different signals emitted by the different components of the device of Figure 3 when this latter runs to implement the method of the invention.

### Detailed description of the invention

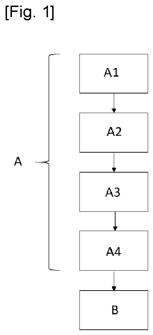

Figure 1 is a block diagram representing the main steps of the method of the invention. In a general way, the method of the invention is a method for estimating a local wavelength of a wave within a sample submitted to a periodic mechanical stress or to a periodic thermal modulation. The wave may be a wave propagating within the sample, a stationary wave or a guided wave such as a Rayleigh or Lamb wave.

This method comprises the following steps:
A) acquiring at least N images of the sample by full-field coherent optical imaging, where N is an integer equal or greater than three,
   for each integer k from 1 to N, acquiring a k^{th} image of the sample while said sample is submitted to said periodic mechanical stress or said periodic thermal modulation as follow:
   A1) launching acquisition of the k^{th} image,
   A2) concurrently to the launching of acquisition of the k^{th} image of step A1), generating a periodic mechanical stress or a periodic thermal modulation, of period T, on the sample,
   A3) with a given time delay τₖ = τ₁ + (k-1)*τ_{offset} with respect to the launching of acquisition of the k^{th} image of step A1) where τ_{offset} is a time offset, illuminating said sample with a series of periodic pulses, of period T, and
   A4) stopping acquisition of the k^{th} image after an acquisition time t_{ACQ} = n*T, where n is an integer greater or equal to 2,
B) estimating the local wavelength by using the N images acquired at step A).

As can be understood, for any image k between 1 and N, the acquisition time t_{ACQ} of the image is defined between the step A1) and the step A4).

The acquisition time t_{ACQ} is advantageously the same for the N images.

Moreover, it is also advantageous to have a periodic launching of image acquisition because it allows for repeatability and thus facilitates wave tracking. In other words, launching of acquisition at steps A1) between the N images is periodic, with a period T₀.

As can also be seen from the method of the invention, a synchronization is made between the light pulses (illumination) and the mechanical stress (in practice, imposed by an actuator that may be, non-limitatively, a vibrator or a piezoelectric) or a thermal modulation (in practice, imposed by a device providing a photothermal effect, for example a modulated light source, the light source being possibly a LED). It means that both signals have a same period T. Accordingly, for the duration t_{ACQ} of an image acquisition, it is the same phase of the periodic signal that is seen.

With regard for example to the approach of De Greef et al., the invention eliminates the need to synchronize all the elements and simply synchronizes the illumination and actuator with each other. In practice, and as mentioned in steps A4), the acquisition is stopped only after a plurality of periods T (plurality of periods = n an integer such that n ≥ 2). In fact, with the method of the invention, the sample is illuminated in periodic movement by several very short synchronized light pulses during a single acquisition time. The number of pulses during an acquisition time is sufficient to fill the pixels of the camera without any powerful light source and/or without any sensitive camera.

From an image (k for example) to another one (k+1 in that example), the offset time t_{offset} gives access to another phase of the periodic signal. The offset time is flexible provided that it is of course greater than the duration of a pulse.

Once step A) implemented, step B) can be implemented to estimate the local wavelength.

Step B) is often qualified as a reconstruction method in the literature.

Many reconstructions methods exist in the literature. For example, we can refer to A. Marmin et al., "Time-of-flight and noise-correlation-inspired algorithms for full-field shear-wave elastography using digital holography", Journal of Biomedical Optics 086006-1 August 2021 • Vol. 26(8), in the chapter "2.3.2 Noise-Correlation-inspired approach". Usually, this reconstruction method is explained from the theory of noise correlation used in seismology which relates the correlation function in space of a random noise field to the focal spot (numerical refocalization) which is directly related to the central (spatial) wavelength. The central wavelength is itself related to the local velocity knowing the central (temporal) frequency. Without entering into mathematic details, this method can be seen as a local wavelength estimator. It is a particularly interesting estimator because the spatial resolution of the wavelength mapping is limited by the resolution of the imaging system (in this paper, the imagining system is based on digital holography) and not by the wavelength of the wave being imaged. It is an essential property for high resolution where the wavelength of the wave is often greater than the field of view provided by the imaging system. It should be noted that if the reconstruction method discussed in this paper is applied to digital holography, the same reconstruction method can of course be applied to any other full-field coherent imaging technique.

In the frame of the invention, we can use an adaptation of the noise-correlation-inspired approach proposed by Marmin et al. In this prior art, the shear-wave (mechanical stress submitted to the sample) is an impulse signal whereas in the present invention, we work with a periodic signal, whatever this latter be mechanical or thermal (step A2): periodic mechanical stress or periodic thermal modulation).

For instance, if we take a sinusoidal signal to represent the periodic mechanical stress or periodic thermal modulation submitted to the sample, it can be represented by s(t) = sin(2π.f.t + φ) with f the signal frequency, t the time and φ a phase. The wave emitted by this source will have the following expression at a local point x within the sample (here in the 1D case, for 2D it's approximately the same thing): O(x,t) = sin(2π.f.t - (2π/λ).x + ϕ) with λ the wavelength of the wave and ϕ a phase.

Firstly, a temporal correlation (over the N images acquired) of a point with its neighbours is made. More precisely, for a point i (at the coordinate xᵢ with our 1D model) in the image, we calculate the correlation C(τ) with the neighbouring point j (at the coordinate xⱼ) (the distance xᵢ to xⱼ is noted dᵢⱼ), we obtain C(τ) = cos(2π.f.τ - (2π/λ).dᵢⱼ).

We can note that the phase ϕ is eliminated.

Secondly, we can derive the correlation function (at least in space, and eventually in time also) thus obtained to recover the local wavelength λ (directly linked to the local speed). Alternatively, we can perform a 2D fit on 3 neighbouring points to estimate λ.

In a more general way, step B) may therefore comprise the following steps:
B1) making a temporal correlation of a point of the sample with its neighbours over the N images acquired at step A,
B2) making a derivation or a 2D fit of the correlation function obtained at step B1) at least in space,
B3) estimating the local wavelength thanks to the results of derivation of step B2.

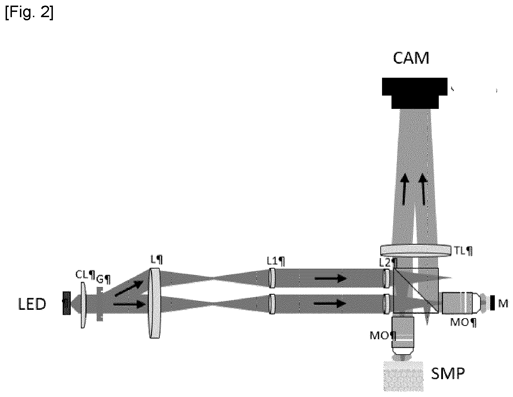

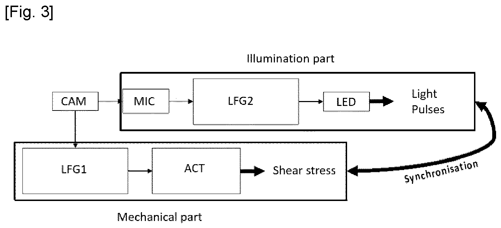

We now describe an example of a device to implement the method of the invention, thanks to figure 2 and figure 3.

In this example and for illustration purposes only, the application of a sinusoidal shear stress on the sample is considered. In such a case, N = 3 images are acquired, recording three phase images of the sinusoidal signal. With these three phase information, it is possible to obtain information about wavelength within the sample during step B). Such a choice also allows a real time treatment of information. To have a good balance between accuracy and time treatment, especially to work in real time, N = 4 image acquisitions may also be considered.

Figure 2 is an example of a full-field optical coherence tomography system (optical part) that can be used for implementing the method of the invention. In figure 2, we can see the Light Emitting Diode LED, the camera CAM and the sample SMP. We can also mention that the optical part of this system also comprises a collimating lens CL, a diffraction grating G, an achromatic lens L and an achromatic afocal device L1, L2. MO designates a microscope objective, M a mirror and TL a tube lens. The black arrows designate the propagation direction of the light emitted by the LED within the system. With this system, interferograms are recorded in one-shot. Direct access to the amplitude and phase image is therefore at the camera's rate, which is suitable for wavefront tracking. The camera may be a 1D camera but preferably, the camera is a 2D camera because it is more adapted to full-field coherent optical imaging.

For more details about the optical system of figure 2, we can refer to the paper of E. Martins Seromenho et al., "Single-shot off-axis full-field optical coherence tomography", Appl. Phys. Lett. 121, 113702 (2022); https://doi.org/10.1063/5.0100944.

In Figure 3, the optical part of Figure 2 is shown within its shear and electronic environment. As can be seen, the camera CAM is connected to both a mechanical part and an illumination part. Advantageously, the camera CAM has its own periodicity T₀ and exposure time t_{ACQ} and in practice therefore sends a same signal at the same time to both the illumination part and the mechanical part to begin acquisition of an image. The camera CAM may be a CCD or a CMOS camera. For example, the camera may be an Adimec Q-2HFW (CMOS camera, 1440x1440 pixels, pixel area = 12 x 12 µm²), that operates at high speed with a maximum acquisition rate of 550 Hz in full frame. The camera is the component that allows implementing step A1) and A4).

In the mechanical part, the actuator ACT may be of the piezoelectric type, for example Cedrat Technologies APA^{®} APA100M that has the ability to reach very high vibration frequencies (up to 100 kHz in sinusoidal mode). The piezoelectric actuator ACT is in contact with the sample SMP (not visible in the figures) and a low-frequency generator LFG1. The low-frequency generator LFG1 is configured to generate the sinusoidal signal of period T. The low-frequency generator LFG1, for example an Agilent 33220A, is finally connected to the camera CAM. The low-frequency-generator and the piezoelectric actuator both contribute to impart a sinusoidal shear stress, of period T, to the sample SMP. The low-frequency generator LFG1 and the actuator ACT are the components that allow implementing step A2).

In the illumination part, the LED may be the ThorLabs M660L4 light-emitting diode (λ₀= 660 nm, FWHM = 20 nm, emitter size = 1.5 x 1.5 mm²) which has the characteristic of being a light source with low spatial and temporal coherence. However, it should be noted that the method of the invention can work with any other wavelength. The LED is powered by a driver (ThorLabs DC2200, not shown) which accepts external modulation signals from a low-frequency generator LFG2, for example Agilent 33220A as for LFG1, to modulate the LED. The low-frequency generator LFG2 is the component that generates the series of periodic pulses at step A3). However, in order to ensure a time delay between the series of pulses (illumination) and the sinusoidal signal (mechanical), a microcontroller MIC, for example Arduino Uno, makes the interface between the camera CAM and the low-frequency generator LFG2. The microcontroller MIC thus controls the time delays τₖ with 1≤ k ≤ N and the offset time τ_{offset} in order to have access to different phases of the sinusoidal signal from an image to another one. The microcontroller MIC, the low-frequency generator LFG2 and the LED are the components that allow implementing step A3).

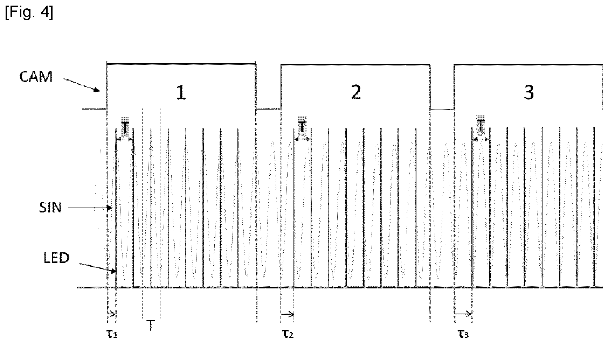

Figure 4 represents the different signals emitted by the different components of the device of Figure 3 when this latter runs to implement the method of the invention.

Once the N = 3 images acquired, they can be used to obtain information on the local wavelength of the mechanical waves within the sample, by implementing step B).

Through this example, we can see that the present method relies solely on the synchronization of the light source and the actuator generating the shear stress (and then a wavefield) on the sample. Contrary to the system of De Greef *et al,* there is no need to synchronize all the components of the system together. Moreover, there is no need for a high-power light source such as a high-power laser to implement the method, a classic LED or a Laser used for imaging are sufficient even though more powerful light sources may alternatively be used as well. In addition, if the present method is finally based on a stroboscopic approach, it is quite different from the classical stroboscopic approach proposed in De Greef *et al.,* as the period T between the light pulses does not impose the acquisition time for an image or conversely.

It is therefore possible to follow the propagation of a field of mechanical waves in enlarged frequency and speed domains, in particular to be capable of analyzing faster waves and/or high-frequency waves within the sample. Typically, measurement with frequencies comprised between and 10 Hz and 100 kHz may be made.

In the case described above with Figures 2 and 3, the optical part of the system to implement the method of the invention is a specific full-field optical coherence tomography system.

However, it must be kept in mind that any full-field coherence tomography system could be considered to implement the method of the invention.

It should also be noted that any other full-field coherent imaging systems, such as any speckle imaging system or any digital holography system could also be used to implement the method of the invention.

Moreover, in the case described above with Figures 2 and 3 control of the acquisition time (t_{ACQ} between steps A1) and A4)) and periodicity (T₀) is carried out by the camera itself. It may be made otherwise however, for example with an additional apparatus sending a control signal to the camera to manage its exposure time and eventually its periodicity.

Further, the conclusions and advantages with regard to the prior art provided here above would be the same for a sinusoidal thermal modulation. More generally, we would also come to the same remarks for any periodic wave (mechanical, thermal modulation), provided that the number N of images acquired is adapted to the frequency information brought by the periodic signal. Indeed, if for a sinusoidal signal, the frequency spectrum is reduced to a single frequency, for other periodic signals, the frequency spectrum is larger. For example, periodically repeated chirps may be used, periodically-repeated gaussians as well. A sinusoidal signal is however preferred as in the case of a non-sinusoidal signal, time analysis is increased.

The invention may be of interest for an application to biological tissues, for example, in order to have accurate and real time analysis of the health of the tissue for a wide range of frequencies and very different kind of tissues (the speed propagation will depend on the properties of the biological tissue analyzed). This method also offers a wide range of possible applications in fields such as biomechanics, industrial testing, and nondestructive testing.

## Claims

1. A method for estimating a local wavelength of a wave within a sample submitted to a periodic mechanical stress or to a periodic thermal modulation, comprising the following steps:
A) acquiring at least N images of the sample by full-field coherent optical imaging, where N is an integer equal or greater than three,
for each integer k from 1 to N, acquiring a k^{th} image of the sample while said sample is submitted to said periodic mechanical stress or said periodic thermal modulation as follow:
A1) launching acquisition of the k^{th} image,
A2) concurrently to the launching of acquisition of the k^{th} image of step A1), generating a periodic mechanical stress or a periodic thermal modulation, of period T, on the sample,
A3) with a given time delay τₖ = τ₁ + (k-1)*τ_{offset} with respect to the launching of acquisition of the k^{th} image of step A1) where τ_{offset} is a time offset, illuminating said sample with a series of periodic pulses, of period T, and
A4) stopping acquisition of the k^{th} image after an acquisition time t_{ACQ} = n*T, where n is an integer greater or equal to 2,
B) estimating the local wavelength by using the N images acquired at step A).

2. The method according to claim 1, wherein an acquisition time (t_{ACQ}) for an image, defined between A4) and A1), is the same for each of the N images.

3. The method according to the preceding claim, wherein launching of acquisition at steps A1) between the N images is periodic, with a period T₀.

4. The method according to one of the preceding claims, wherein the mechanical stress or the thermal modulation is sinusoidal.

5. The method according to the preceding claim, wherein step A) is carried out for N = 3 or 4 images.

6. The method according to claim 4 or 5, wherein the mechanical stress is a shear stress.

7. The method according to one of the preceding claims, wherein step B) comprises the following steps:
B1) make a temporal correlation of a point of the sample with its neighbours over the N images acquired at step A,
B2) make a derivation or a fit of the correlation function obtained at step B1) at least in space,
B3) estimating the local wavelength thanks to the results of derivation or fit of step B2).

8. The method according to one of the preceding claims, wherein the wave is a wave propagating within the sample, a stationary wave or a guided wave such as a Rayleigh or Lamb wave.

9. The method according to one of the preceding claims, wherein acquisition of the N images is carried out with a 2D camera (CAM), for example of CMOS type.

10. The method according to the preceding claim, wherein the camera (CAM) has its own image acquisition time (t_{ACQ}), and preferably its own periodicity (T₀).

11. The method according to one of the preceding claims, wherein the periodic mechanical stress is generated by a piezoelectric actuator (ACT) in contact with the sample together with a first low-frequency generator (LFG1) connected to said piezoelectric actuator (ACT) and configured to generate a sinusoidal signal of period T.

12. The method according to claims 9 and 10, wherein the low-frequency generator (LFG1) is connected to the camera (CAM).

13. The method according to one of the preceding claims, wherein illumination is carried out by a Light-Emitting Diode (LED).

14. The method according to the preceding claim, wherein the series of periodic pulses is generated by a second low-frequency generator (LFG2) connected to the Light-Emitting Diode (LED).

15. The method according to claims 9 and 14, wherein the time delays are generated by a microcontroller (MIC) both connected to the camera (CAM) and to the second low-frequency generator (LFG2).
